(19) Europäisches Patentamt
European Patent Office
Office européen des brevets

(11) Veröffentlichungsnummer: **0 316 843 B1**

(12) # EUROPÄISCHE PATENTSCHRIFT

(45) Veröffentlichungstag der Patentschrift: **02.03.94**

(51) Int. Cl.⁵: **C07D 487/04**, A01N 43/90, //(C07D487/04,239:00,239:00), (C07D487/04,239:00,235:00)

(21) Anmeldenummer: **88118955.9**

(22) Anmeldetag: **14.11.88**

(54) **1,2,3,4-Tetrahydro-5-nitro-pyrimidin-Derivate.**

(30) Priorität: **18.11.87 DE 3739264**

(43) Veröffentlichungstag der Anmeldung:
**24.05.89 Patentblatt 89/21**

(45) Bekanntmachung des Hinweises auf die Patenterteilung:
**02.03.94 Patentblatt 94/09**

(84) Benannte Vertragsstaaten:
**BE CH DE FR GB IT LI NL**

(56) Entgegenhaltungen:
**EP-A- 0 247 477**
**US-A- 4 031 087**

(73) Patentinhaber: **BAYER AG**

**D-51368 Leverkusen(DE)**

(72) Erfinder: **Gesing, Ernst, Dr.**
**Trillser Graben 4**
**D-4006 Erkrath-Hochdahl(DE)**
Erfinder: **Wolf, Hilmar, Dr.**
**Haus Gravener Strasse 105**
**D-4018 Langenfeld(DE)**
Erfinder: **Becker, Benedikt, Dr.**
**Metzkausener Strasse 14**
**D-4020 Mettmann(DE)**
Erfinder: **Homeyer, Bernhard, Dr.**
**Obere Strasse 28**
**D-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue 1,2,3,4-Tetrahydro-5-nitro-pyrimidin-Derivate, ein Verfahren zu ihrer Herstellung und ihre Verwendung in Schädlingsbekämpfungsmitteln, insbesondere als Insektizide.

Es ist bereits bekannt, daß bestimmte Pyrimidino-thiazine wie z. B. 7-Ethyl-9-nitro-3,4,7,8-tetrahydro-(2H,6H)-pyrimidino-[4,3-b]-1,3-thiazin insektizide Eigenschaften aufweisen (vergl. US-PS 4 031 087). 1,2,3,4-Tetrahydro-5-nitro-pyrimidin-Derivate sind aus EP-A-247477 (Publiziert am 02-12-87) bekannt.

Es wurden nun die neuen 1,2,3,4-Tetrahydro-5-nitro-pyrimidin-Derivate der Formel (I),

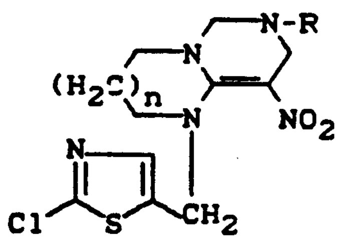

(I)

in welcher

- n für die Zahlen 0 oder 1 steht und
- R für die Gruppe $-A-R^1$ steht, in welcher
- A für eine direkte Bindung oder für die Gruppierungen $-(CH_2)_m-$ oder $-(CH_2)_x-Y-(CH_2)_z$ steht, worin
- m für die Zahlen 1 bis 4 steht,
- x und z für die Zahlen 0, 1 oder 2 stehen, wobei x und z gleich oder verschieden sein können und
- Y für Sauerstoff, Schwefel oder für die Gruppierungen -NH- oder

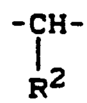

steht, worin

- $R^2$ für gegebenenfalls durch Alkoxycarbonyl substituiertes $C_1$-$C_4$-Alkyl, Cyano, Hydroxy oder für Phenyl steht und
- $R^1$ für Halogen-$C_1$-$C_4$-alkyl oder für gegebenenfalls substituierte Reste aus der Reihe Phenyl, Naphthyl, Anthryl, Phenanthryl, Pyridyl, Furyl, Thienyl, Piperidyl oder 1,3-Benzodioxolyl steht,

und deren Säureadditions-Salze gefunden.

Einige der neuen 1,2,3,4-Tetrahydro-5-nitro-pyrimidin-Derivate der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man die 1,2,3,4-Tetrahydro-5-nitro-pyrimidin-Derivate der Formel (I) und deren Säureadditions-Salze erhält, wenn man Nitromethylen-Derivate der Formel (II)

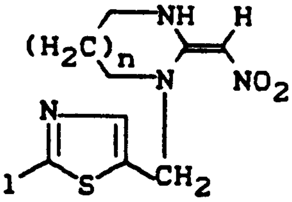

(II)

in welcher

- n die oben angegebene Bedeutung hat,

mit Aminen der Formel (III)

$R^1$-A-$NH_2$ (III)

in welcher

$R^1$ und A die oben angegebene Bedeutung haben,

in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen physiologisch verträgliche Säuren addiert.

Überraschenderweise zeichnen sich die erfindungsgemäßen 1,2,3,4-Tetrahydro-5-nitro-pyrimidin-Derviate der Formel (I) in überragender Weise durch eine hohe Wirksamkeit als Insektizide aus.

Die Erfindung betrifft vorzugsweise Verbindungen der Formel (I), in welcher

n für die Zahlen 0 oder 1 steht,

R für die Gruppe -A-$R^1$ steht,
in welcher

A für die Gruppierungen -$(CH_2)_m$- oder -$(CH_2)_x$-Y-$(CH_2)_z$ steht,
worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

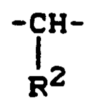

steht,
worin

$R^2$ für gegebenenfalls durch $C_1$-$C_4$-Alkoxycarbonyl substituiertes $C_1$-$C_4$-Alkyl, Cyano, Hydroxy oder für Phenyl steht und

$R^1$ für Halogen-$C_1$-$C_4$-alkyl oder für gegebenenfalls einfach bis fünffach, gleich oder verschieden substituierte Reste aus der Reihe Phenyl, Naphthyl, Anthryl, Phenanthryl, Pyridyl, Furyl, Thienyl, Piperidyl oder 1,3-Benzodioxolyl steht, wobei als Substituenten genannt seien: Halogen, $C_1$-$C_4$-Alkyl, Halogen-$C_1$-$C_2$-alkyl, $C_1$-$C_2$-Alkoxy, Halogen-$C_1$-$C_2$-alkylthio, Hydroxy, Di-$C_1$-$C_2$-alkylamino, Carboxyl und Phenyl.

Besonders bevorzugt sind diejenigen Verbindungen der Formel (I) in welcher

n für die Zahlen 0 oder 1 steht,

R für die Gruppe -A-$R^1$ steht,
in welcher

A für die Gruppierungen -$(CH_2)_m$- oder -$(CH_2)_x$-Y-$(CH_2)_z$ steht,
worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

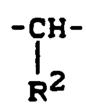

steht,
worin

$R^2$ für gegebenenfalls durch Alkoxycarbonyl substituiertes $C_1$-$C_4$-Alkyl, Cyano, Hydroxy oder für Phenyl steht und

$R^1$ für Halogen-$C_1$-$C_4$-alkyl oder für gegebenenfalls einfach bis fünffach, gleich oder verschieden substituierte Reste aus der Reihe Phenyl, Naphthyl, Anthryl, Phenanthryl, Pyridyl, Furyl, Thienyl, Piperidyl oder 1,3-Benzodioxolyl steht, wobei als Substituenten genannt seien: Fluor, Chlor, Brom, Methyl, Ethyl, n-oder i-Propyl, tert.-Butyl, Trifluormethyl, Methoxy, Ethoxy,

Trifluormethylthio, Hydroxy, Dimethylamino, Diethylamino, Carboxyl und Phenyl.

Bevorzugte erfindungsgemäße Verbindungen sind auch Additionsprodukte aus Säuren und denjenigen 1,2,3,4-Tetrahydro-5-nitro-pyrimidin-Derivaten der Formel (I), in denen die Substituenten R, $R^1$, $R^2$ oder der Index n die Bedeutungen haben, die bereits vorzugsweise für diese Substituenten und den Index genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Verwendet man beispielsweise für das erfindungsgemäße Verfahren 3-(2-Chlor-1,3-thiadiazol-5-yl-methyl)-2-nitromethylen-imidazolidin, 4-Fluorbenzylamin und mindestens die zweifache molare Menge Formaldehyd als Ausgangsstoffe, so kann die entsprechende Reaktion durch das folgende Formelschema wiedergegeben werden:

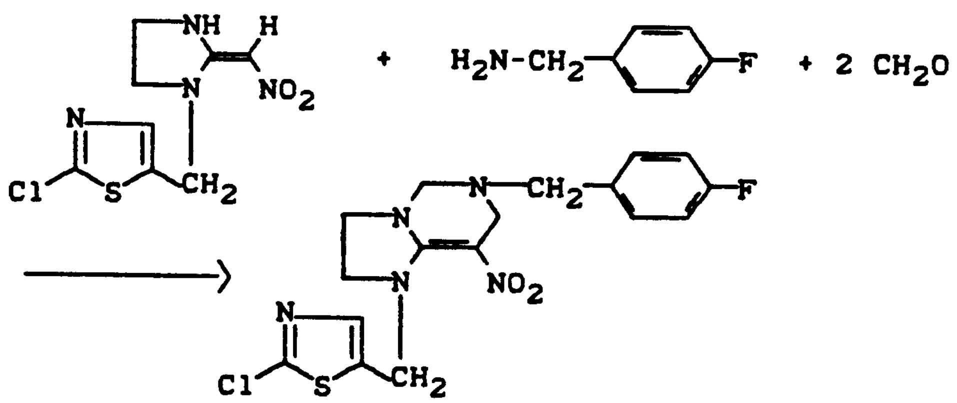

Die beim erfindungsgemäßen Verfahren als Ausgangsstoffe zu verwendenden Nitromethylen-Derivate sind durch die Formel (II) allgemein definiert. In dieser Formel (II) hat der Index n vorzugsweise diejenige Bedeutung, die bereits im Zusammenhang mit der Beschreibung der erfindungsgemäßen Stoffe der Formel (I) vorzugsweise für diesen Index n genannt wurde.

Die Verbindungen der Formel (II) sind bekannt und/oder lassen sich nach bekannten Methoden herstellen( vergl. z. B. DE-OS 2 514 402, EP-OS 136 636, EP-OS 154 178 und EP-OS 163 855).

Die beim erfindungsgemäßen Verfahren außerdem als Ausgangsstoffe zu verwendenden Amine sind durch die Formel (III) allgemein definiert. In dieser Formel (III) stehen $R^1$ und A vorzugsweise für diejenigen Reste, die bereits im Zusammenhang mit der Beschreibung der erfindungsgemäßen Stoffe der Formel (I) vorzugsweise für diese Substituenten genannt wurden.

Die Amine der Formel (III) sind allgemein bekannte Verbindungen der organischen Chemie.

Als Beispiele für die Verbindungen der Formel (III) seien genannt:

$R^1$-A-$NH_2$ (III)

**Tabelle 1**

| A | $R^1$ |
|---|---|
| $-\overset{\overset{CH_3}{\vert}}{C}H-$ | 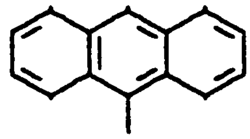 |
| $-\overset{\overset{CH_3}{\vert}}{C}H-$ | 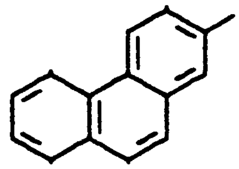 |
| $-\overset{\overset{CH_3}{\vert}}{C}H-$ | 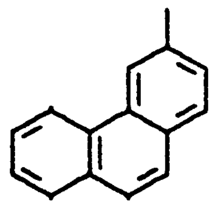 |
| $-\overset{\overset{CH_3}{\vert}}{C}H-$ | 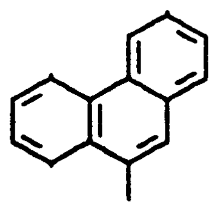 |
| $-CH_2-$ | 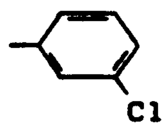 |
| $-CH_2-$ | 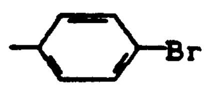 |

Tabelle 1 - Fortsetzung

| A | $R^1$ |
|---|---|
| $-CH_2-$ | 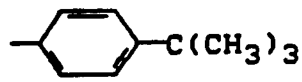 |
| $-CH_2-$ | 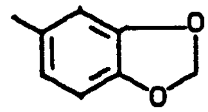 |
| $-CH_2-$ | 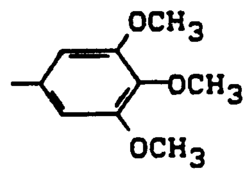 |
| $-CH_2-$ | 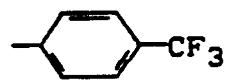 |
| $-CH_2-$ | 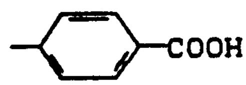 |
| $-CH_2-$ | 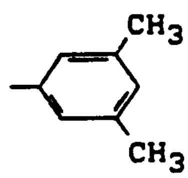 |
| $-CH_2-$ | 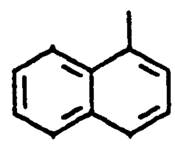 |
| $-CH_2-CH_2-$ | 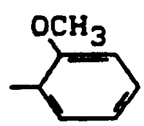 |
| $-CH_2-CH_2-$ | 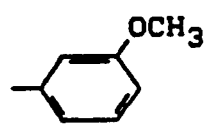 |

**Tabelle 1** - Fortsetzung

| A | $R^1$ |
|---|---|
| $-CH_2-CH_2-$ | 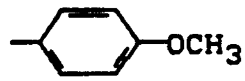 |
| $-CH_2-CH_2-$ | 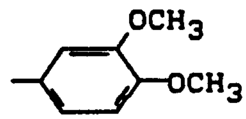 |
| $-CH_2-CH_2-$ | 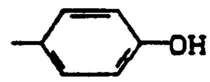 |
| $-CH_2-CH_2-$ | 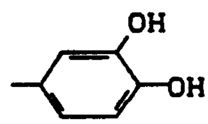 |
| $-CH_2-CH_2-$ | 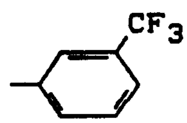 |
| $-CH(OH)-CH_2-$ | 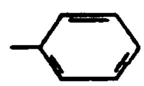 |
| $-(CH_2)_3-$ | 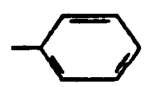 |
| $-CH(CH_3)-(CH_2)_2-$ | 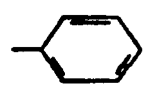 |
| $-(CH_2)_4-$ | 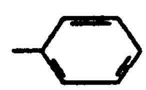 |

**Tabelle 1** - Fortsetzung

| A | $R^1$ |
|---|---|
| 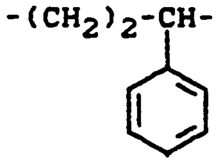 | 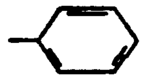 |
| 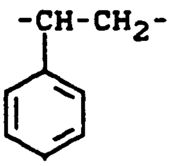 | 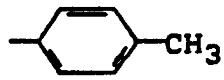 |
| 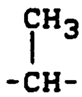 | 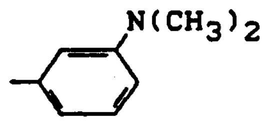 |
| 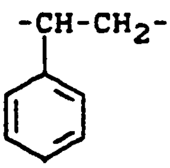 | 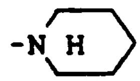 |
| 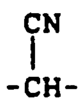 | 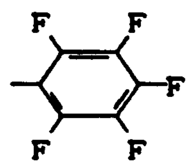 |
| 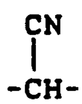 | 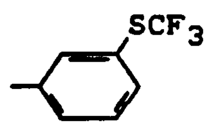 |
| 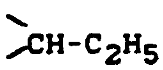 | 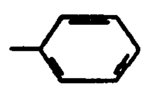 |

Tabelle 1 - Fortsetzung

| A | $R^1$ |
|---|---|
| 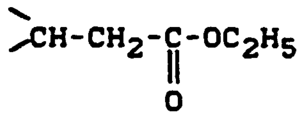 | 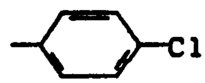 |
| 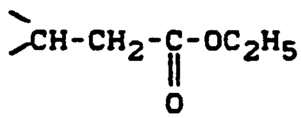 | 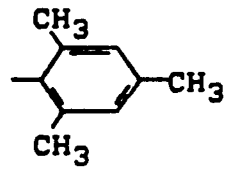 |
| 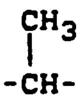 | 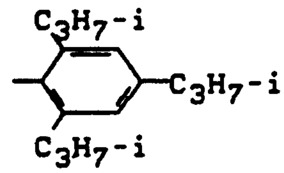 |

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesetzt.

Das erfindungsgemäße Verfahren wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zwischen -20 °C und +120 °C, vorzugsweise bei Temperaturen zwischen 0 °C und +80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingesetzt. Die Reaktionen werden im allgemeinen in einem geeigneten Verdünungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben

erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa ssp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.

Die erfindungsgemäßen Wirkstoffe der Formel (I) zeichnen sich durch eine hervorragende insektizide Wirksamkeit aus. Sie zeigen insbesondere beim Einsatz als Blattinsektizide und Bodeninsektizide eine hervorragende Wirkung gegen Maden wie z.B. Phorbia antiqua-Maden gegen Raupen, wie z. B. Plutella maculipennis, gegen Käferlarven, wie z.B. Phaedon cochleariae und Diabrotica balteata und Blattläuse, wie z.B. Myzus persicae und Aphis fabae.

Die neuen Verbindungen sind also besonders gut für einen Einsatz zur Bekämpfung von Blattinsekten und Bodeninsekten geeignet.

Weiterhin zeigen die neuen Verbindungen eine bakterizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthtische Stoffe, Feinstverkapselungen in polymeren Stoffen und in Hüllmassen für Saatgut, ferner in Formulierungen

mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage; z.B. nichtionogene und anionische Emulgatoren, wie Polyoxyethylen-Fettsäure-Ester, Polyoxyethylen-Fettalkohol-Ether, z.B. Alkylarlypolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäße Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können. Sie zeigen insbesondere beim Einsatz als Ektoparasitizide eine ausgezeichnete Wirkung gegen Blowfly-larven wie z. B. Lucilia cuprina.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden.

Herstellungsbeispiel

Beispiel 1

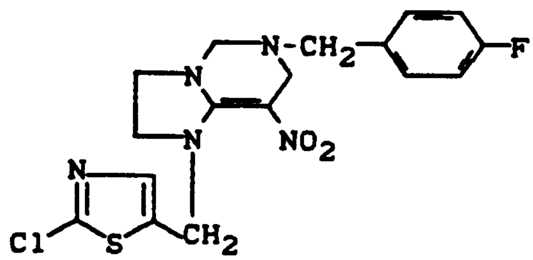

Zu einer Mischung aus 2,6 g (0,01 Mol) 3-(2-Chlor-1,3-thiadiazol-5-yl-methyl)-2-nitromethylen-imidazolidin und 1,25 g (0,01 Mol) 4-Fluorbenzylamin in 90 ml Ethanol werden bei Raumtemperatur 1,5 ml (0,02 Mol) 37%-ige wäßrige Formaledehydlösung zugetropft und 3 Stunden unter Rückfluß erhitzt. Nach dem Abkühlen auf Raumtemperatur wird das Lösungsmittel im Vakuum entfernt, der Rückstand mit Ether versetzt und abgesaugt.

Man erhält 3,4 g (83 % der Theorie) 6,7-Dihydro-6-(4-fluorbenzyl)-8-nitro-(5H)-3-(2-chlor-1,3-thiadiazol-5-yl-methyl)-imidazolidino-[2,3-f]-pyrimidin vom Schmelzpunkt 178 °C.

Analog Beispiel 1 bzw. dem erfindungsgemäßen Verfahren können die in der nachfolgenden Tabelle 2 angegebenen Verbindungen der Formel (I) hergestellt werden:

Tabelle 2:

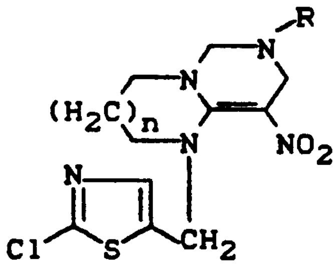

(I)

| Bsp.Nr. | n | R | physikalische Konstante |
|---|---|---|---|
| 2 | 1 | 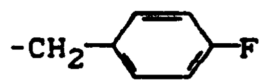 | Fp: 167° C |
| 3 | 1 | 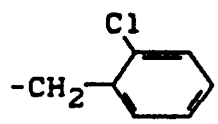 | Fp:117° C |
| 4 | 0 | 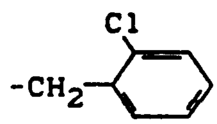 | Fp:156° C |
| 5 | 0 | 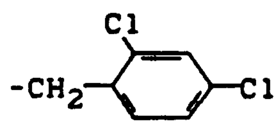 | Fp:162° C |
| 6 | 1 | 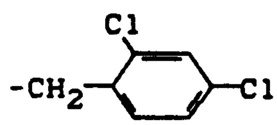 | Fp:98° C |
| 7 | 1 | 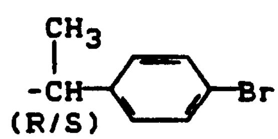 | Fp:154° C |
| 8 | 1 | 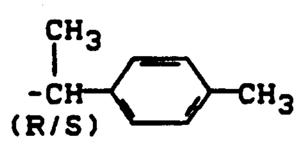 | Fp:139° C |

**Tabelle 2** - Fortsetzung

| Bsp.Nr. | n | R | physikalische Konstante |
|---|---|---|---|
| 9 | 1 | 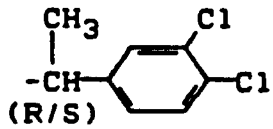 | gelbes viskoses Öl |
| 10 | 1 | 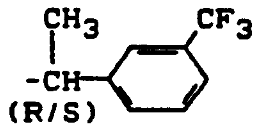 | Fp:91° C |
| 11 | 1 | 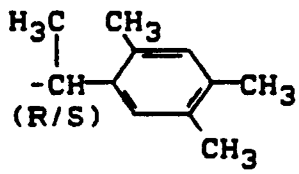 | Fp:157° C |
| 12 | 1 | 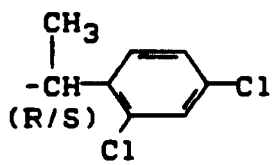 | Fp:94° C |
| 13 | 0 | 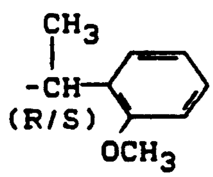 | Fp:157° C |
| 14 | 0 | 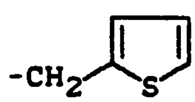 | Fp:141° C |
| 15 | 0 | 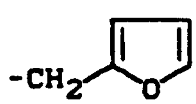 | Fp:119° C |
| 16 | 0 | 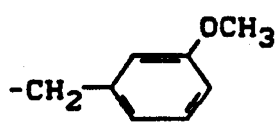 | Fp:139° C |

Tabelle 2 - Fortsetzung

| Bsp.Nr. | n | R | physikalische Konstante |
|---|---|---|---|
| 17 | 0 | 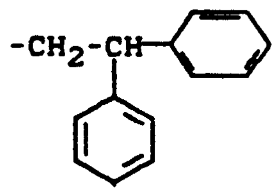 | Fp: 194° C |
| 18 | 0 | 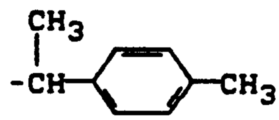 | Fp: 109° C |
| 19 | 0 | 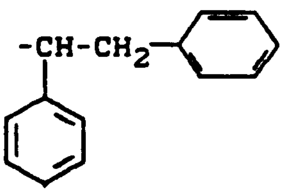 | Fp: 164° C |
| 20 | 0 | 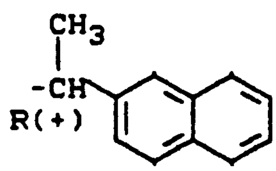 | Fp: 94° C |
| 21 | 1 | 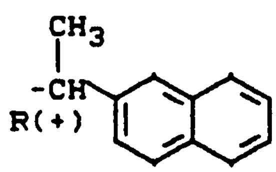 | Fp: 78° C |
| 22 | 1 | 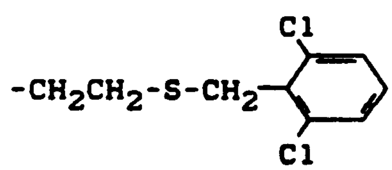 | Fp: 91° C |
| 23 | 1 | 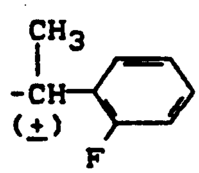 | Fp: 142° C |

Tabelle 2 - Fortsetzung

| Bsp.Nr. | n | R | physikalische Konstante |
|---|---|---|---|
| 24 | 1 | 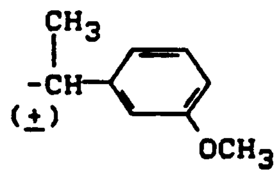 | Fp:88° C |
| 25 | 1 | 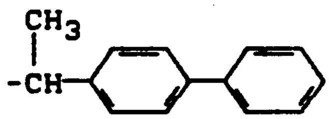 | Fp:76° C |
| 26 | 1 | 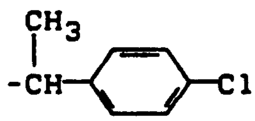 | Fp:167° C |
| 27 | 1 | 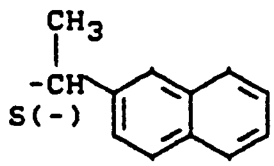 | Fp:81° C |
| 28 | 0 | 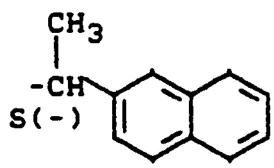 | Fp:89° C |
| 29 | 0 | $-CH_2-CF_3$ | Fp:113° C |
| 30 | 0 | 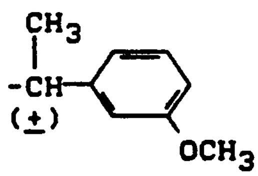 | Fp:150° C |
| 31 | 0 | $-CH_2CH_2-S-CH_2-$ 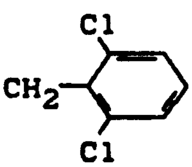 | Fp:139° C |

**Tabelle 2** - Fortsetzung

| Bsp.Nr. | n | R | physikalische Konstante |
|---|---|---|---|
| 32 | 0 | 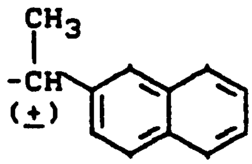 | Fp:173° C |
| 33 | 0 | 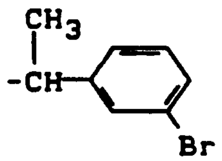 | Fp:154° C |
| 34 | 0 | 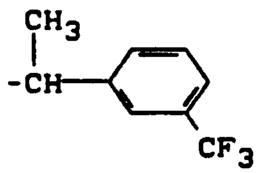 | Fp:112° C |
| 35 | 0 | 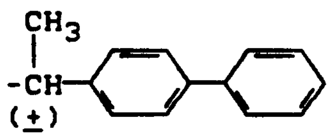 | Fp:153° C |
| 36 | 0 | 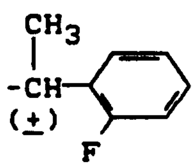 | Fp:197° C |
| 37 | 1 | 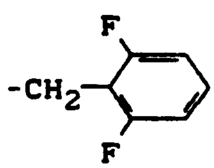 | Fp:171° C |
| 38 | 0 | 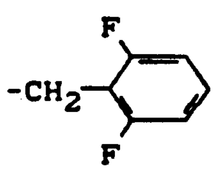 | Fp:159° C |
| 39 | 0 | 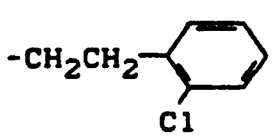 | Fp:147° C |

Tabelle 2 - Fortsetzung

| Bsp.Nr. | n | R | physikalische Konstante |
|---|---|---|---|
| 40 | 1 | 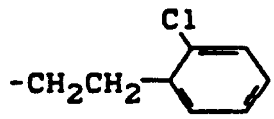 | Fp: 91° C |
| 41 | 0 | 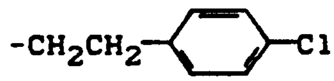 | Fp: 131° C |
| 42 | 0 | 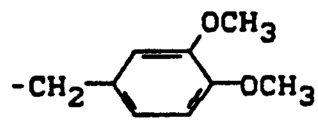 | Fp: 145° C |
| 43 | 1 | 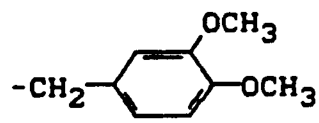 | Fp: 177° C |
| 44 | 0 | 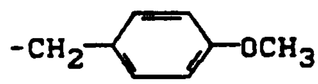 | Fp: 152° C |
| 45 | 1 | 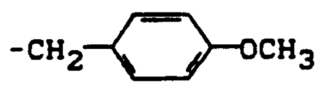 | Fp: 140° C |
| 46 | 0 | 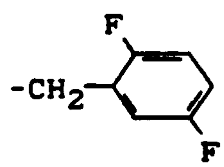 | Fp: 162° C |
| 47 | 1 | 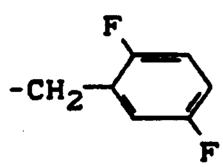 | Fp: 126° C |
| 48 | 1 | 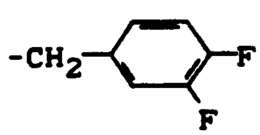 | Fp: 158° C |

Tabelle 2 - Fortsetzung

| Bsp.Nr. | n | R | physikalische Konstante |
|---|---|---|---|
| 49 | 0 | 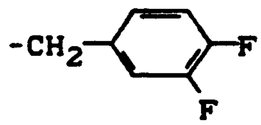 | Fp:160° C |
| 50 | 1 | 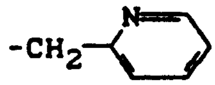 | Fp:127° C |
| 51 | 0 | 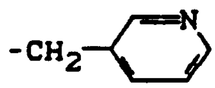 | Fp: 58° C |
| 52 | 1 | 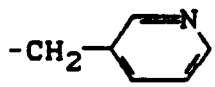 | Fp:137° C |
| 53 | 0 | 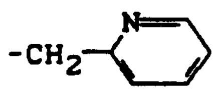 | Fp:149° C |
| 54 | 0 | 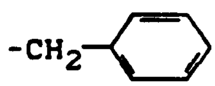 | Fp:159° C |
| 55 | 1 | 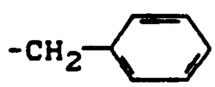 | Fp:126° C |
| 56 | 1 | 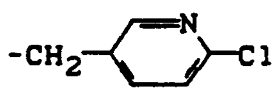 | Fp:134° C |
| 57 | 0 | 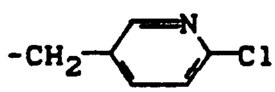 | Fp:126° C |
| 58 | 1 | 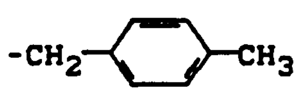 | Fp:147° C |

Tabelle 2 - Fortsetzung

| Bsp.Nr. | n | R | physikalische Konstante |
|---|---|---|---|
| 59 | 1 | 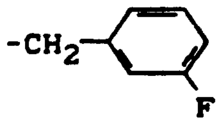 | Fp:119° C |
| 60 | 0 | 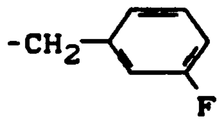 | Fp:115° C |
| 61 | 0 | 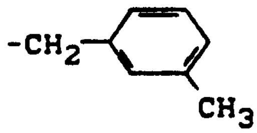 | Fp:127° C |
| 62 | 1 | 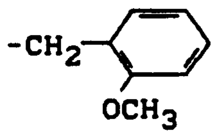 | Fp:133° C |
| 63 | 0 | 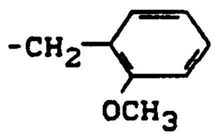 | Fp:161° C |
| 64 | 1 | 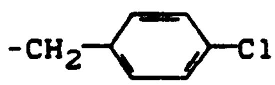 | Fp:141° C |
| 65 | 0 | 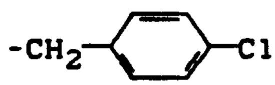 | Fp:181° C |
| 66 | 0 | 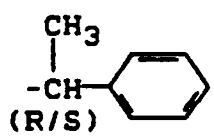 | Fp:169° C |
| 67 | 1 | 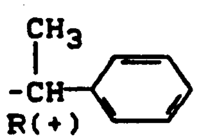 | Fp:177° C |

Tabelle 2 - Fortsetzung

| Bsp.Nr. | n | R | physikalische Konstante |
|---|---|---|---|
| 68 | 1 | 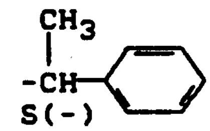 | Fp:161° C |
| 69 | 1 | 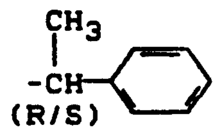 | Fp:149° C |
| 70 | 0 | $-CH_2CH_2-O-CH_2-CF_3$ | Fp:89° C |
| 71 | 0 | 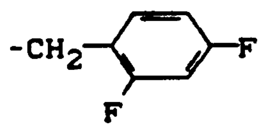 | Fp:152° C |
| 72 | 0 | 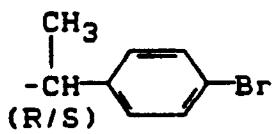 | Fp:78° C |
| 73 | 0 | 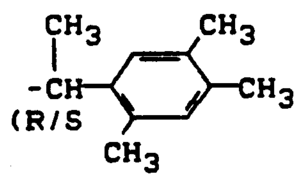 | Fp:183° C |
| 74 | 1 | 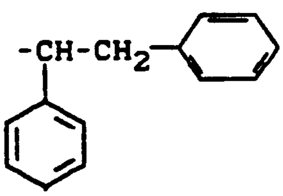 | Fp:121° C |
| 75 | 1 | 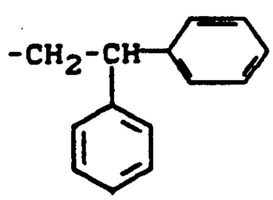 | Fp:196° C |

Tabelle 2 - Fortsetzung

| Bsp.Nr. | n | R | physikalische Konstante |
|---|---|---|---|
| 76 | 1 | 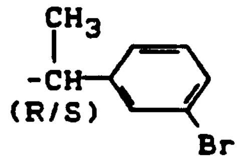 | Fp:131° C |
| 77 | 1 | 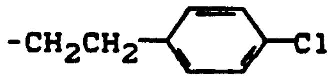 | Fp:161° C |
| 78 | 1 | 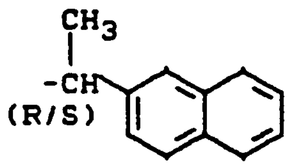 | Fp:79° C |
| 79 | 1 | 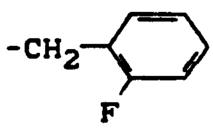 | Fp:91° C |
| 80 | 0 | 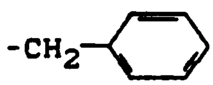 | Fp:167° C |
| 81 | 0 | 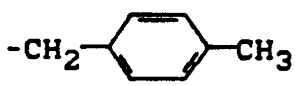 | Fp:149° C |
| 82 | 1 | 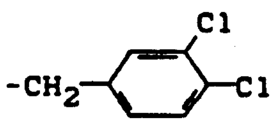 | Fp:167° C |
| 83 | 0 | 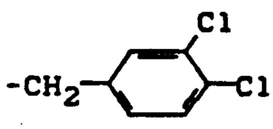 | Fp:147° C |

Tabelle 2 - Fortsetzung

| Bsp.Nr. | n | R | physikalische Konstante |
|---|---|---|---|
| 84 | o | 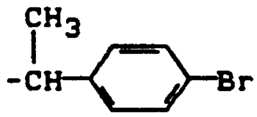 | Fp:114° C |
| 85 | O | 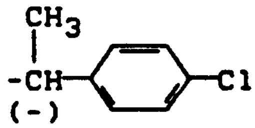 | Fp:113° C |
| 86 | 1 | 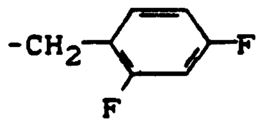 | Fp:109° C |
| 87 | 1 | 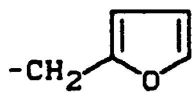 | Fp:136° C |
| 88 | 1 | 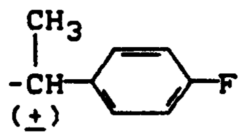 | Fp:106° C |
| 89 | 1 | 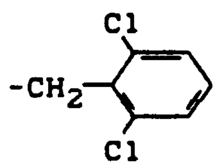 | Fp:158° C |
| 90 | 1 | 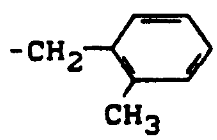 | Fp:134° C |
| 91 | 1 | 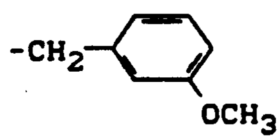 | Fp:171° C |
| 92 | 1 | 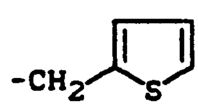 | Fp:83° C |

**Tabelle 2 - Fortsetzung**

| Bsp.Nr. | n | R | physikalische Konstante |
|---|---|---|---|
| 93 | 0 | 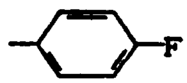 | Fp:148° C |
| 94 | 0 | 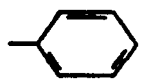 | Fp:177° C |

Verwendungsbeispiele

In den folgenden Verwendungsbeispielen wurde die folgende Verbindung als Vergleichssubstanz eingesetzt:
(A)

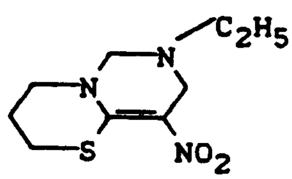

7-Ethyl-9-nitro-3,4,7,8-tetrahydro-(2H, 6H)-pyrimidino-[4,3-b]-1,3-thiazin aus US-PS 4 031 087.

Beispiel A

Phaedon-Larven-Test

Lösungsmittel: 3 Gewichtsteile Dimethylformamid
Emulgator: 1 Gewichtsteil Alkylarlypolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z.B. die folgenden Verbindungen der Herstellungsbeispiele 80, 79, 67, 68, 69, 2, 1, 3, 4, 5, 6, 82, 81, 65, 64, 62, 63, 61, 60, 59, 58, 57, 56, 54, 55, 53, 50, 51, 52, 49, 48, 47, 44, 45, 41, 42, 43, 39, 40, 38, 37, 35, 34, 33, 32, 29, 30, 31, 28, 27, 26, 23, 24, 25, 20, 21, 13, 76, 77, 78, 7, 8, 9, 10, 11, 12, 74, 73, 71, 72, 70, 86, 87, 85 und 84 überlegene Wirksamkeit gegenüber dem Stand der Technik.

Beispiel B

Aphis-Test (systemische Wirkung)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Aphis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele 80, 66, 3, 4, 62, 63, 60, 49, 44, 41, 42, 39, 34, 33, 29, 13, 76, 7, 8, 10, 11, 74 und 73 überlegene Wirksamkeit gegenüber dem Stand der Technik.

Beispiel C

Grenzkonzentrations-Test / Wurzelsystemische Wirkung

| | |
|---|---|
| Testinsekt: | Myzus persicae |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele 63 und 39 überlegene Wirkung genenüber dem Stand der Technik.

Beispiel D

Grenzkonzentrations-Test / Bodeninsekten

| | |
|---|---|
| Testinsekt: | Phorbia antiqua-Maden im Boden |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt zum Beispiel die folgende Verbindung der Herstellungsbeispiele 63 überlegene Wirksamkeit gegenüber dem Stand der Technik.

## Patentansprüche

**1.** 1,2,3,4-Tetrahydro-5-nitro-pyrimidin-Derivate der Formel (I)

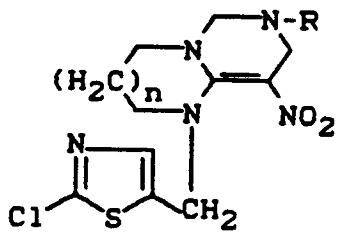

(I)

in welcher

n für die Zahlen 0 oder 1 steht und

R für die Gruppe -A-$R^1$ steht in welcher

A für eine direkte Bindung oder für die Gruppierungen $-(CH_2)_m-$ oder $-(CH_2)_x-Y-(CH_2)_z$ steht, worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

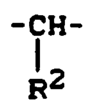

steht, worin

$R^2$ für gegebenenfalls durch Alkoxycarbonyl substituiertes $C_1$-$C_4$-Alkyl, Cyano, Hydroxy oder für Phenyl steht und $R^1$ für Halogen-$C_1$-$C_4$-alkyl oder für gegebenenfalls substituierte Reste aus der Reihe Phenyl, Naphthyl, Anthryl, Phenanthryl, Pyridyl, Furyl, Thienyl, Piperidyl oder 1,3-Benzodioxolyl steht,

und deren Säureadditionssalze.

**2.** 1,2,3,4-Tetrahydro-5-nitro-pyrimidin-Derivate der Formel (I) gemäß Anspruch 1, in welcher

n für die Zahlen 0 oder 1 steht,

R für die Gruppe -A-$R^1$ steht,

in welcher

A für die Gruppierungen $-(CH_2)_m-$ oder $-(CH_2)_x-Y-(CH_2)_2$ steht, worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

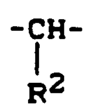

steht,
worin

$R^2$ für gegebenenfalls durch $C_1$-$C_4$-Alkoxycarbonyl substituiertes $C_1$-$C_4$-Alkyl, Cyano, Hydroxy oder für Phenyl steht und

$R^1$ für Halogen-$C_1$-$C_4$-alkyl oder für gegebenenfalls einfach bis fünffach, gleich oder verschieden substituierte Reste aus der Reihe Phenyl, Naphthyl, Anthryl, Phenanthryl, Pyridyl, Furyl, Thienyl, Piperidyl oder 1,3-Benzodioxolyl steht.

**3.** 1,2,3,4-Tetrahydro-5-nitro-pyrimidin-Derivate der Formel (I) gemäß Anspruch 1, in welcher

n für die Zahlen 0 oder 1 steht,

R fur die Gruppe -A-$R^1$ steht,
in welcher

A für die Gruppierungen $-(CH_2)_m-$ oder $-(CH_2)_x-Y-(CH_2)_z$ steht,
worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

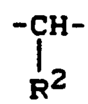

steht,
worin

$R^2$ für gegebenenfalls durch Alkoxycarbonyl substituiertes $C_1$-$C_4$-Alkyl, Cyano, Hydroxy oder für Phenyl steht und

$R^1$ für Halogen-$C_1$-$C_4$-alkyl oder für gegebenenfalls einfach bis fünffach, gleich oder verschieden substituierte Reste aus der Reihe Phenyl, Naphthyl, Anthryl, Phenanthryl, Pyridyl, Furyl, Thienyl, Piperidyl oder 1,3-Benzodioxolyl steht.

**4.** Verfahren zur Herstellung von 1,2,3,4-Tetrahydro-5-nitro-pyrimidin-Derivaten der Formel (I)

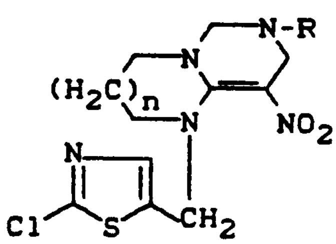

(I)

in welcher

n für die Zahlen 0 oder 1 steht und

R für die Gruppe -A-$R^1$ steht,
in welcher

A für eine direkte Bindung oder für die Gruppierungen $-(CH_2)_m-$ oder $-(CH_2)_x-Y-(CH_2)_z$ steht,
worin

m für die Zahlen 1 bis 4 steht,

x und z für die Zahlen 0, 1 oder 2 stehen, wobei x und z gleich oder verschieden sein können und

Y für Sauerstoff, Schwefel oder für die Gruppierungen -NH- oder

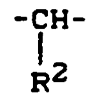

steht,
worin

$R^2$ für gegebenenfalls durch Alkoxycarbonyl substituiertes $C_1$-$C_4$-Alkyl, Cyano, Hydroxy oder für Phenyl steht und

$R^1$ für Halogen-$C_1$-$C_4$-alkyl oder für gegebenenfalls substituierte Reste aus der Reihe Phenyl, Naphthyl, Anthryl, Phenanthryl, Pyridyl, Furyl, Thienyl, Piperidyl oder 1,3-Benzodioxolyl steht,

und von deren Säureadditionssalzen,
dadurch gekennzeichnet, daß man Nitromethylen-Derivate der Formel (II)

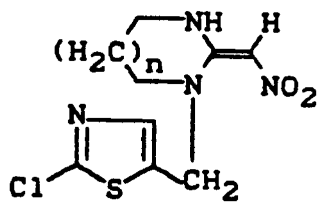

(II)

in welcher
n die oben angegebene Bedeutung hat,
mit Aminen der Formel (III)

$R^1$-A-$NH_2$ (III)

in welcher
$R^1$ und A die oben angegebene Bedeutung haben,
in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen Säuren addiert.

**5.** Schädlingsbekämpfungsmittel, gekennzeichnet durch einen Gehalt an mindestens einem 1,2,3,4-Tetrahydro 5-nitropyrmidin-Derivat der Formel (I).

**6.** Insektizide Mittel gekennzeichnet durch einen Gehalt an mindestens einem 1,2,3,4-Tetrahydro-5-nitro-pyrimidin-Derivat der Formel (I).

**7.** Verfahren zur Bekämpfung von Insekten, dadurch gekennzeichnet, daß man ein 1,2,3,4-Tetrahydro-5-nitro-pyrimidin-Derivat der Formel (I) auf Insekten und/oder deren Lebensraum einwirken läßt.

**8.** Verwendung von 1,2,3,4-Tetrahydro-5-nitro-pyrimidin-Derivaten der Formel (1) zur Bekämpfung von Insekten.

**9.** Verfahren zur Herstellung von Schädlingsbekämpfungsmitteln, dadurch gekennzeichnet, daß man 1,2,3,4-Tetrhydro-5-nitropyrimidin-Derivate der Formel (I) mit Streckmitteln und/oder oberflächenaktiven Mitteln vermischt.

## Claims

**1.** 1,2,3,4-Tetrahydro-5-nitro-pyrimidine derivatives of the formula (I)

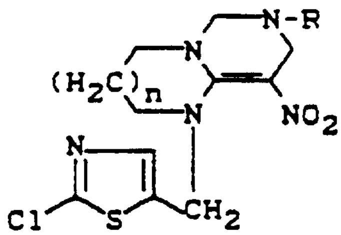

(I)

in which

n represents the numbers 0 or 1 and

R represents the -A-$R^1$ group,
in which

A represents a direct bond or the $-(CH_2)_m-$ or $-(CH_2)_x-Y-(CH_2)_z$ groupings,
where

m represents the numbers 1 to 4,

x and z represent the numbers 0, 1 or 2, it being possible for x and z to be identical or different, and

Y represents oxygen, sulphur or the -NH- or

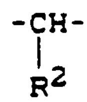

groupings,
where

$R^2$ represents optionally alkoxycarbonyl-substituted $C_1$-$C_4$-alkyl, cyano, hydroxyl or phenyl and

$R^1$ represents halogeno-$C_1$-$C_4$-alkyl or optionally substituted radicals from the series comprising phenyl, naphthyl, anthryl, phenanthryl, pyridyl, furyl, thienyl, piperidyl and 1,3-benzodioxolyl,

and their acid addition salts.

**2.** 1,2,3,4-Tetrahydro-5-nitro-pyrimidine derivatives of the formula (I) according to Claim 1
in which

n represents the numbers 0 or 1,

R represents the -A-$R^1$ group,

in which

A represents the $-(CH_2)_m$ or $-(CH_2)_x-Y-(CH_2)_z$ groupings,
where

m represents the numbers 1 to 4,

x and z represent the numbers 0, 1 or 2, it being possible for x and z to be identical or different, and

Y represents oxygen, sulphur or the -NH- or

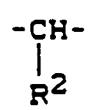

groupings,
where

$R^2$ represents optionally $C_1$-$C_4$-alkoxycarbonyl-substituted $C_1$-$C_4$-alkyl, cyano, hydroxyl or phenyl and

$R^1$ represents halogeno-$C_1$-$C_4$-alkyl or radicals from the series comprising phenyl, naphthyl, anthryl, phenanthryl, pyridyl, furyl, thienyl, piperidyl and 1,3-benzodioxolyl which are optionally mono-substituted to pentasubstituted by identical or different substituents.

**3.** 1,2,3,4-Tetrahydro-5-nitro-pyrimidine derivatives of the formula (I) according to Claim 1, in which

n represents the numbers 0 or 1,

R represents the -A-$R^1$ group, in which

A represents the $-(CH_2)_m-$ or $-(CH_2)_x-Y-(CH_2)_z-$groupings, where

m represents the numbers 1 to 4,

x and z represent the numbers 0, 1 or 2, it being possible for x and z to be identical or different, and

Y represents oxygen, sulphur or the -NH- or

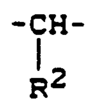

groupings, where

$R^2$ represents optionally alkoxycarbonyl-substituted $C_1$-$C_4$-alkyl, cyano, hydroxyl or phenyl and

$R^1$ represents halogeno-$C_1$-$C_4$-alkyl or radicals from the series comprising phenyl, naphthyl, anthryl, phenanthryl, pyridyl, furyl, thienyl, piperidyl and 1,3-benzodioxolyl which are optionally mono-substituted to pentasubstituted by identical or different substituents.

**4.** Process for the preparation of 1,2,3,4-tetrahydro-5-nitro-pyrimidine derivatives of the formula (I)

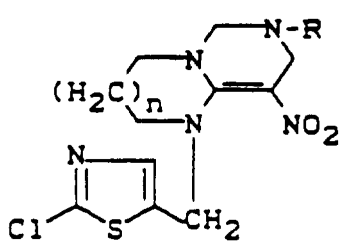

(I)

in which

n represents the numbers 0 or 1,

R represents the -A-$R^1$ group, in which

A represents a direct bond or the $-(CH_2)_m-$ or $-(CH_2)_x-Y-(CH_2)_z-$groupings, where

m represents the numbers 1 to 4,

x and z represent the numbers 0, 1 or 2, it being possible for x and z to be identical or different, and

Y represents oxygen, sulphur or the -NH- or

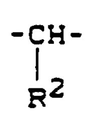

groupings,
where

$R^2$ represents optionally alkoxycarbonyl-substituted $C_1$-$C_4$-alkyl, cyano, hydroxyl or phenyl and

$R^1$ represents halogeno-$C_1$-$C_4$-alkyl or optionally substituted radicals from the series comprising phenyl, naphthyl, anthryl, phenanthryl, pyridyl, furyl, thienyl, piperidyl and 1,3-benzodioxolyl,

and of their acid addition salts,
characterized in that nitromethylene derivatives of the formula (II)

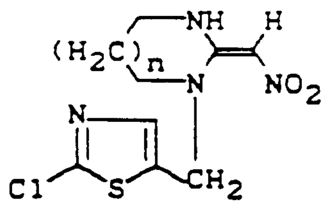

(II)

in which
n has the abovementioned meaning,
are reacted with the amines of the formula (III)

$R^1$-A-$NH_2$ (III)

in which
$R^1$ and A have the abovementioned meaning in the presence of at least twice the molar amount of formaldehyde, if appropriate in the presence of acid catalysts and if appropriate in the presence of diluents, and, if appropriate, the resulting compounds are subjected to an addition reaction with acids.

5. Pesticides, characterized in that they contain at least one 1,2,3,4-tetrahydro-5-nitropyrimidine derivative of the formula (I).

6. Insecticidal agents, characterized in that they contain at least one 1,2,3,4-tetrahydro-5-nitro-pyrimidine derivative of the formula (I).

7. Method combating insects, characterized in that a 1,2,3,4-tetrahydro-5-nitro-pyrimidine derivative of the formula (I) is allowed to act on insects and/or their habitat.

8. Use of 1,2,3,4-tetrahydro-5-nitro-pyrimidine derivatives of the formula (I) for combating insects.

9. Process for the preparation of pesticides, characterized in that 1,2,3,4-tetrahydro-5-nitropyrimidine derivatives of the formula (I) are mixed with extenders and/or surface active agents.

**Revendications**

1. Dérivés de la 1,2,3,4-tétrahydro-5-nitropyrimidine de formule (I):

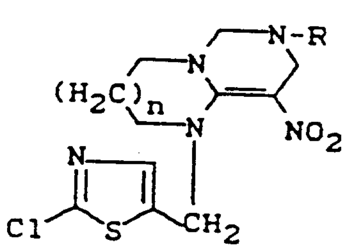

(I)

dans laquelle

n représente les nombres 0 ou 1, et

R représente le groupe $-A-R^1$, dans lequel

A représente une liaison directe ou les groupes $-(CH_2)_m-$ ou $-(CH_2)_x-Y-(CH_2)_z$, dans lesquels

m représente les nombres 1 à 4,

x et z représentent les nombres 0, 1 ou 2, x et z pouvant être identiques ou différents, et

Y représente l'oxygène, le soufre ou les groupes -NH- ou

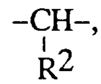

où

$R^2$ représente un groupe alkyle en $C_1$-$C_4$ éventuellement alcoxycarbonyle-substitué, cyano, hydroxy ou phényle, et

$R^1$ représente un groupe halogénoalkyle en $C_1$-$C_4$ ou des restes éventuellement substitués de la série des restes phényle, naphtyle, anthryle, phénanthryle, pyridyle, furyle, thiényle, pipéridyle ou 1,3-benzodioxolyle,

et leurs sels d'addition d'acide.

**2.** Dérivés de la 1,2,3,4-tétrahydro-5-nitropyrimidine de formule (I) selon la revendication 1, dans laquelle

n représente les nombres 0 ou 1, et

R représente le groupe $-A-R^1$, dans lequel

A représente les groupes $-(CH_2)_m-$ ou $-(CH_2)_x-Y-(CH_2)_z$, dans lesquels

m représente les nombres 1 à 4,

x et z représentent les nombres 0, 1 ou 2, x et z pouvant être identiques ou différents, et

Y représente l'oxygène, le soufre ou les groupes -NH- ou

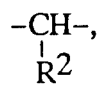

où

$R^2$ représente un groupe alkyle en $C_1$-$C_4$ éventuellement substitué par un groupe alcoxycarbonyle en $C_1$-$C_4$, cyano, hydroxy ou phényle, et

$R^1$ représente un groupe halogénoalkyle en $C_1$-$C_4$ ou des restes éventuellement substitués une à cinq fois de manière identique ou différente, de la série des restes phényle, naphtyle, anthryle, phénanthryle, pyridyle, furyle, thiényle, pipéridyle ou 1,3-benzodioxolyle.

**3.** Dérivés de la 1,2,3,4-tétrahydro-5-nitropyrimidine de formule (I) selon la revendication 1, dans laquelle

n représente les nombres 0 ou 1, et

R représente le groupe $-A-R^1$, dans lequel

A représente les groupes $-(CH_2)_m-$ ou $-(CH_2)_x-Y-(CH_2)_z$, dans lesquels

m représente les nombres 1 à 4,

x et z représentent les nombres 0, 1 ou 2, x et z pouvant être identiques ou différents, et

Y représente l'oxygène, le soufre ou les groupes -NH- ou

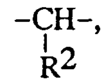

où

R² représente un groupe alkyle en $C_1$-$C_4$ éventuellement alcoxycarbonyle-substitué, cyano, hydroxy ou phényle, et

R¹ représente un groupe halogénoalkyle en $C_1$-$C_4$ ou des restes éventuellement substitués une à cinq fois de manière identique ou différente, de la série des restes phényle, naphtyle, anthryle, phénanthryle, pyridyle, furyle, thiényle, pipéridyle ou 1,3-benzodioxolyle.

**4.** Procédé de préparation de dérivés de la 1,2,3,4-tétrahydro-5-nitro-pyrimidine de formule (I)

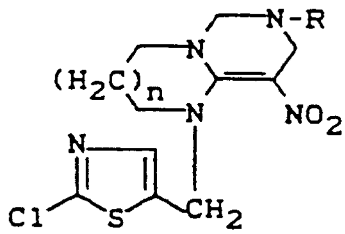

(I)

dans laquelle

n représente les nombres 0 ou 1, et

R représente le groupe -A-$R^1$, dans lequel

A représente une liaison directe ou les groupes $-(CH_2)_m-$ ou $-(CH_2)_x-Y-(CH_2)_z$, dans lesquels

m représente les nombres 1 à 4,

x et z représentent les nombres 0, 1 ou 2, x et z pouvant être identiques ou différents, et

Y représente l'oxygène, le soufre ou les groupes -NH- ou

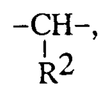

où

$R^2$ représente un groupe alkyle en $C_1$-$C_4$ éventuellement alcoxycarbonyle-substitué, cyano, hydroxy ou phényle, et

$R^1$ représente un groupe halogénoalkyle en $C_1$-$C_4$ ou des restes éventuellement substitués de la série des restes phényle, naphtyle, anthryle, phénanthryle, pyridyle, furyle, thiényle, pipéridyle ou 1,3-berizodioxolyle,

et de leurs sels d'addition d'acide,

caractérisé en ce que l'on fait réagir des dérivés de nitrométhylène de formule (II):

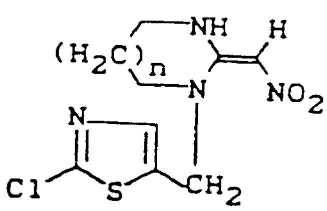

(II)

dans laquelle

n a la signifieation indiquée ci-dessus,
avec des amines de formule (III)

$R^1$-A-$NH_2$ (III)

dans laquelle
$R^1$ et A ont la signification indiquée ci-dessus,
en présence d'au moins la quantité deux fois molaire de formaldéhyde, éventuellement en présence de catalyseurs acides et éventuellement en présence de diluants, et on ajoute éventuellement des acides aux composés obtenus.

**5.** Agents de lutte contre les nuisibles, caractérisés par une teneur en au moins un dérivé de la 1,2,3,4-tétrahydro-5-nitropyrimidine de formule (I).

**6.** Agents insecticides, caractérisés par une teneur en au moins un dérivé de la 1,2,3,4-tétrahydro-5-nitropyrimidine de formule (I).

**7.** Procédé de lutte contre les insectes, caractérisé en ce que l'on fait agir sur les insectes et/ou sur leur biotope un dérivé de la 1,2,3,4-tétrahydro-5-nitropyrimidine de formule (I).

**8.** Utilisation de dérivés de la 1,2,3,4-tétrahydro-5-nitropyrimidine de formule (I) pour la lutte contre les insectes.

**9.** Procédé de préparation d'agents de lutte contre les nuisibles, caractérisé en ce que l'on mélange des dérivés de la 1,2,3,4-tétrahydro-5-nitropyrimidine de formule (I) avec des diluants et/ou des agents tensioactifs.